# FASCICULE DE BREVET EUROPEEN

(11) **EP 2 146 708 B1**
(45) Date de publication et mention de la délivrance du brevet: **15.10.2014**
(21) Numéro de dépôt: 07803968.2
(22) Date de dépôt: 29.06.2007
(51) Int. Cl.: A61K 31/375, A61K 31/52, A61K 36/73, A61K 36/752, A61K 36/77

(54) **COMPOSITION A ACTIVITÉ LIPOLYTIQUE, SON PROCÉDÉ D'OBTENTION ET UTILISATION DE LA COMPOSITION.**
ZUSAMMENSETZUNG MIT LIPOLYTISCHER AKTIVITÄT, HERSTELLUNGSVERFAHREN DAFÜR UND VERWENDUNG DER ZUSAMMENSETZUNG
COMPOSITION HAVING LIPOLYTIC ACTIVITY, PRODUCTION METHOD THEREOF AND USE OF THE COMPOSITION.

(30) Priorité: 04.05.2007 FR 0754856
(43) Date de publication de la demande: 27.01.2010
(73) Titulaire: Fytexia, 34350 Vendres (FR)
(72) Inventeur: DALLAS, Constantin, F-34500 Beziers (FR)
(74) Mandataire: Rhein, Alain
(86) Numéro de dépôt international: PCT/FR2007/051559
(87) Numéro de publication internationale: WO 2008/135643

(56) Documents cités:
- WO-A-2005/067952
- US-A1- 2002 058 075
- US-A1- 2006 099 277
- US-A1- 2006 204 599
- US-B1- 6 340 482
- US-B1- 6 565 847
- US-B1- 6 576 272
- COLKER C M ET AL: "EFFECTS OF CITRUS AURANTIUM EXTRACT, CAFFEINE, AND ST.JOHN'S WORT ON BODY FAT LOSS, LIPID LEVELS, AND MOOD STATES IN OVERWEIGHT HEALTHY ADULTS" CURRENT THERAPEUTIC RESEARCH, vol. 60, no. 3, mars 1999 (1999-03), pages 145-153, XP000957650
- DULLOO A G ET AL: "Efficacy of a green tea extract rich in catechin polyphenols and caffeine in increasing 24-h energy expenditure and fat oxidation in humans" AMERICAN JOURNAL OF CLINICAL NUTRITION, BETHESDA,MD, US, vol. 70, no. 6, décembre 1999 (1999-12), pages 1040-1045, XP001154724 ISSN: 0002-9165

## Description

L'invention concerne une composition à activité lipolytique, notamment obtenue à partir d'agrumes.

L'invention concerne encore un procédé pour l'obtention d'une telle composition ainsi que l'utilisation d'une telle composition.

L'invention entre dans le domaine des compléments alimentaires et des poudres pour leur application alimentaire, c'est-à-dire notamment leur incorporation dans des aliments.

La lipolyse est le phénomène biologique correspondant à la dégradation des graisses par l'organisme.

Une telle dégradation peut notamment permettre de libérer l'énergie stockée sous forme de graisses dans le tissu adipeux. Au niveau physiologique, cette dégradation intervient notamment au niveau des cellules adipocytaires, sous forme d'une réaction consistant à libérer des acides gras et du glycérol par hydrolyse des triglycérides stockés dans lesdites cellules adipocytaires.

Ainsi, favoriser le phénomène de lipolyse est une solution efficace pour encourager la perte de masse corporelle et le raffermissement corporel assisté par l'exercice musculaire, dans la mesure où la lipolyse conduit au brûlage des graisses et peut ainsi permettre d'augmenter les performances physiques.

Certains compléments alimentaires sont connus pour leur action dans ce sens, mais ceux-ci incorporent de la synéphrine, un alcaloïde structurellement apparenté à l'éphédrine. Des effets indésirables sur les plans cardiovasculaire et vasculaire cérébral ont été rapportés suite à l'usage de compositions comprenant ces molécules.

D'autres molécules sont également connues pour leur activité potentiellement, mais non exclusivement, lipolytique. Il s'agit par exemple de la théophylline, de la caféine, ou encore de l'isoprotérénol. Si la théophylline et la caféine sont largement connues pour être retrouvées naturellement dans des plantes comme le thé ou le café, ce sont également des substances excitantes dont l'excès n'est pas compatible avec un régime alimentaire sain et équilibré. L'isoprotérénol est une substance médicamenteuse.

La présente invention se veut à même de répondre aux attentes des consommateurs recherchant un complément alimentaire ou poudre pour son application alimentaire à activité lipolytique, d'origine naturelle, efficace tout en restant exempt de tout effet secondaire indésirable.

Des recherches menées sur des extraits de fruits, notamment d'agrumes, ont permis de mettre en évidence l'action lypolitique unique d'une composition selon l'invention.

A cet effet, l'invention concerne une composition à activité lipolytique comprenant des polyphénols, notamment sous forme de flavonoïdes, en particulier des flavanones et des anthocyanines, et d'acides phénoliques, et de la caféine, lesdits polyphénols étant représentés par au moins 0,1 à 30% de flavanones, 0,01 à 10% d'anthocyanines et 0,01 à 5% d'acides phénoliques, les pourcentages se référant à la composition totale et le total de polyphénols présents atteignant au moins 30% de la composition totale, caractérisée en ce qu'elle est obtenue à partir d'un mélange d'espèces végétales comportant au moins 5 à 30% en poids d'oranges sanguines (*Citrus sinensis L. Osbeck),* 5 à 30% en poids de pamplemousses (*Citrus paradisi* Macfad.), 5 à 25% en poids d'oranges douces (*Citrus sinensis L. Osbeck),* 1 à 10% en poids d'oranges amères (*Citrus aurantium L.* ), 5 à 20% en poids de Guarana (*Paullinia cupana Kunth*) et en ce que ladite composition est exempte de synéphrine

La composition selon l'invention peut encore comprendre en plus de la vitamine C.

Selon une autre caractéristique de l'invention, la composition selon l'invention comprend entre 60 et 90% de polyphénols.

Selon une autre caractéristique de l'invention, la composition selon l'invention comprend 0,1 à 4% de caféine.

Selon une autre caractéristique de l'invention, la composition selon l'invention comprend 0,1 à 3% de vitamine C.

Avantageusement, la composition selon l'invention se présente, seule ou avec des adjuvants supplémentaires, sous forme d'une poudre hydrosoluble ou non. Cette poudre peut être intégrée à différentes formes galéniques ou employée seule dans des produits alimentaires.

L'invention concerne encore un procédé pour l'obtention d'une telle composition selon l'invention, caractérisé par le fait qu'il comporte, à partir d'un mélange d'espèces végétales comportant au moins 5 à 30% en poids d'oranges sanguines *(Citrus sinensis L. Osbeck),* 5 à 30% en poids de pamplemousses *(Citrus paradisi* Macfad.), 5 à 25% en poids d'oranges douces (*Citrus sinensis L. Osbeck*), 1 à 10% en poids d'oranges amères (*Citrus aurantium L.*), 5 à 20% en poids de Guarana (*Paullinia cupana Kunth*), au moins :
- une étape d'extraction de polyphénols de jus d'orange sanguine *(Citrus sinensis L. Osbeck)* et d'orange douce *(Citrus sinensis L. Osbeck),*
- une étape d'extraction de polyphénols de sous-produits d'orange sanguine (*Citrus sinensis L. Osbeck),* de pamplemousse (*Citrus paradisi Macfad.*), d'orange douce (*Citrus sinensis L. Osbeck)* et d'orange amère (*Citrus* aurantium *L.*),
- une étape de mélange des extraits obtenus des étapes d'extraction précédentes.
- puis enfin une étape de tamisage du produit résultant afin d'obtenir une poudre.

Selon une autre caractéristique de l'invention, le procédé est caractérisé par le fait que l'on procède à l'extraction des anthocyanines et acides phénoliques de jus d'orange sanguine *(Citrus sinensis L. Osbeck)* et d'orange douce *(Citrus sinensis L. Osbeck*) et, à l'extraction de flavanones de sous-produits d'orange sanguine (*Citrus sinensis L. Osbeck*), de pamplemousse *(Citrus paradisi* Macfad.), d'orange douce *(Citrus sinensis L. Osbeck)* et d'orange amère (*Citrus* aurantium *L.*).
- suite à l'étape de mélange, une étape de séchage du mélange par atomisation sur un support maltodextrine,
- suite à l'étape de séchage, une étape de normalisation en caféine, notamment par l'ajout d'un extrait de Guarana (*Paullinia cupana Kunth*), et/ou de Mate (*Ilex Paraguariensis* A.St.Hil), et/ou de Kola (*Cola Nitida* (Vent.) Schott & Endl.), au produit résultant de l'étape de séchage,

L'invention concerne encore l'utilisation de la composition selon l'invention comme complément alimentaire ou pour enrichir les aliments, notamment les boissons telles que les jus de fruits et/ou légumes ainsi que les produits lactés.

Avantageusement, la composition selon l'invention est utilisée pour son administration en vue de favoriser le brûlage des graisses ou favoriser la perte de masse corporelle.

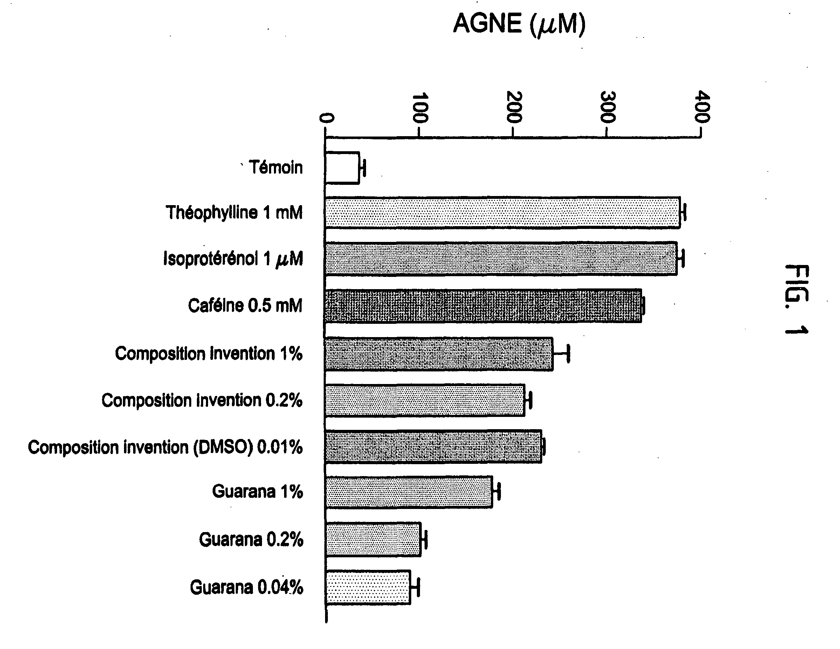

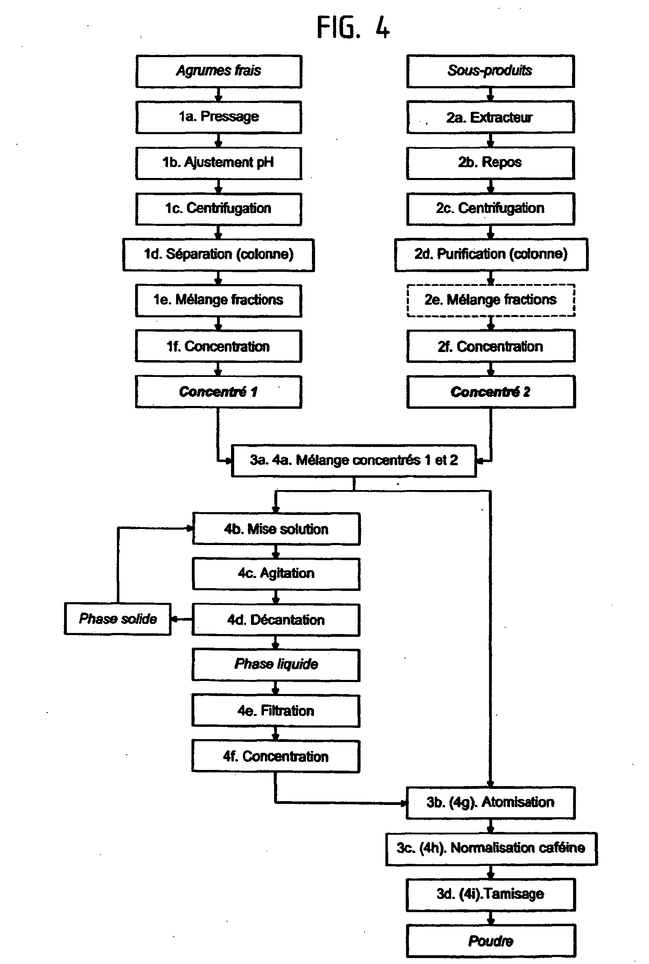

Les buts et avantages de la présente invention apparaîtront à description détaillée qui va suivre se rapportant à plusieurs exemples de réalisation. La compréhension de cette description sera facilitée en se référant aux dessins ci-joints, dans lesquels les figures 1 à 3 présentent des résultats expérimentaux attestant l'efficacité de la composition selon l'invention, et la figure 4 un diagramme illustrant un mode d'exécution du procédé pour l'obtention de la composition selon l'invention.

La présente invention concerne le domaine des compléments alimentaires et des poudres pour leur application alimentaire, c'est-à-dire notamment leur incorporation dans des aliments, et a trait, plus particulièrement, à une composition à activité lipolytique, notamment obtenue à partir d'agrumes.

Les espèces végétales pour son obtention ont été retenues de manière à obtenir une composition apportant un juste dosage de substances actives agissant en synergie.

En particulier, la composition selon l'invention tire parti d'une synergie entre les effets des polyphénols, notamment sous forme de flavonoïdes et d'acides phénoliques, et de la caféine qu'elle contient.

Avantageusement, la synergie est augmentée par la présence en plus de vitamine C.

Les polyphénols sont plus précisément retrouvés sous forme de flavonoïdes et d'acides phénoliques. Plus particulièrement, la composition selon l'invention présente des teneurs particulières en flavanones et anthocyanines, qui sont deux sous-groupes des flavonoïdes, ainsi que des teneurs particulières d'acides phénoliques.

Le tableau 1 présente le dosage de ces différentes substances actives essentielles dans un mode de réalisation préféré de la composition selon l'invention.

Plus particulièrement, la composition à activité lipolytique selon l'invention comprend au moins 30% de polyphénols, préférentiellement entre 60 et 90%, et 0,1 à 4% de caféine.

Les polyphénols sont plus particulièrement représentés par des flavanones, des anthocyanines et des acides phénoliques. Avantageusement la composition comprend plus particulièrement 0,1 à 30% de flavanones, 0,01 à 10% d'anthocyanines et 0,01 à 5% d'acides phénoliques, les pourcentages se référant à la composition totale et le total de polyphénols présents atteignant au moins 30% de la composition totale.

Les flavanones peuvent se présenter, non exclusivement ni exhaustivement, mais de façon dominante, par exemple sous forme d'hespéridine, de naringine, d'isonaringine, de narirutine, de neohespéridine, de naringenine.

Les anthocyanines peuvent se présenter, non exclusivement ni exhaustivement, mais de façon dominante, par exemple sous forme de cyanidine-3-glucoside, de malvidine-3-glucoside, de peonidine-3-glucoside, de petunidine-3-glucoside.

Les acides phénoliques peuvent se présenter, non exclusivement ni exhaustivement, mais de façon dominante, par exemple sous forme d'acide caféique, d'acide coumarique, d'acide férulique, d'acide cinammique, d'acide sinapique.

La vitamine C est avantageusement présente à hauteur de 0,1 à 3% de la composition totale.

Les pourcentages sont calculés à partir de l'analyse d'un échantillon de la composition selon l'invention au travers de diverses méthodes. Les polyphénols totaux sont dosés à l'aide de la spectrophotométrie Ultra-Violet à 280 nm, et les anthocyanines à l'aide de la même technique à 550 nm. Les flavanones, acides phénoliques, également les anthocyanines, la caféine et la vitamine C sont quant à eux dosés par Chromatographie Liquide Haute Performance (HPLC).

Un procédé optimisé que nous décrirons par ensuite permet d'obtenir la composition à activité lipolytique selon l'invention, qui se présente au final sous forme d'une poudre, hydrosoluble ou non.

De façon surprenante, des expérimentations ont reconnu le dosage en substances actives présenté plus haut comme particulièrement efficace pour répondre aux objectifs visés, à savoir un effet sur le brûlage des graisses et ainsi sur l'augmentation des performances physiques.

Tout particulièrement, la composition selon l'invention agit sur la lipolyse.

Pour une meilleure compréhension de ces expérimentations, il est nécessaire d'exposer au préalable les facteurs biologiques en jeu dans la lipolyse.

La lipolyse est une réaction consistant à libérer des acides gras et du glycérol par hydrolyse de lipides. Ceci peut intervenir au niveau de la digestion des lipides alimentaires mais aussi au niveau du tissu adipeux, où la réaction de lipolyse périphérique consiste à libérer des acides gras et du glycérol par hydrolyse de triglycérides stockés dans les cellules adipocytaires de l'organisme. C'est cette réaction qui nous intéresse tout particulièrement, correspondant à l'effet attendu de brûlage de graisses stockées.

La lipolyse est une réaction hormonosensible, notamment aux hormones « catécholamines » comme l'adrénaline ou la noradrénaline. Elle est enclenchée par la liaison de ces hormones sur les récepteurs β-3 des cellules adipocytaires, ce qui entraîne une activation de l'adényl-cyclase, enzyme de la synthèse d'AMP cyclique (cAMP), qui conduit ainsi à une augmentation intracellulaire d'AMP cyclique (cAMP). L'AMP cyclique (cAMP) est une molécule activatrice de la triglycéride lipase, elle-même responsable de la libération des graisses de réserve stockées sous forme de triglycérides dans les cellules adipocytaires. La libération des graisses de réserve se fait par hydrolyse des triglycérides, selon la réaction :

1 triglycéride → 3 Acides Gras Libres (AGL) + 1 Glycérol

Les AGL libérés servent ensuite de substrat énergétique aux cellules de l'organisme.

L'inhibition de la lipolyse, qui induit l'effet inverse de celui attendu, peut notamment être conduite par l'action de deux enzymes : la cathéchol-o-methyl transférase et la phosphodiestérase. La cathéchol-o-methyl transferase intervient dans le catabolisme des hormones venant se lier aux récepteurs P-3, et conduit à limiter la stimulation du cycle conduisant à la libération d'AGL. La phosphodiestérase intervient dans la dégradation de l'AMP cyclique (cAMP) en une forme non cyclique inactive, ce qui conduit également à limiter la stimulation du cycle conduisant à la libération d'AGL.

Les expérimentations qui ont été conduites afin de tester l'efficacité de la composition selon l'invention consistent d'une part en une expérience ex-vivo réalisée sur des adipocytes humains, dans le but d'évaluer les effets de la composition selon l'invention sur la libération d'AGL encore appelés Acides Gras Non-Estérifiés (AGNE), ceci comparativement à d'autres substances, et d'autre part en une expérience in-vivo, c'est-à-dire une étude clinique réalisée sur des sujets en surpoids auxquels la composition selon l'invention a été administrée, comparativement à l'administration d'un placebo.

Ces deux expériences sont particulièrement révélatrices des effets physiologiques d'une administration de la composition selon l'invention.

### EXPERIENCE 1

Une première expérience dont les résultats figurent en tableau 2 et figure 1 visait la détection des effets d'une administration de la composition selon l'invention sur la libération d'Acides Gras Non-Estérifiés (AGNE) par des adipocytes humains maintenus en suspension. En effet, cette mesure est révélatrice de l'effet de la composition selon l'invention sur l'hydrolyse des triglycérides adipocytaires.

Cette expérience a été menée sur des adipocytes humains isolés à partir d'une plastie abdominale d'un donneur de sexe féminin, âgé de 35 ans. Dès réception, les fragments de tissus adipeux ont été incubés pendant 30 minutes à 37°C en présence de collagénase. Les adipocytes isolés et lavés ont été repris avec 4 volumes de milieu d'essai (bicarbonate 1,87 mg/mL, pénicilline/streptomycine 25 UI/mL/25µg/mL, glutamine 2mM, MEM sans rouge phénol qsq 100% (v/v), additionné d'albumine bovine sérique délipidée à 0,5% (p/v)).

Dans le but d'obtenir des résultats comparatifs, différents composés ont été testés en parallèle sur les adipocytes en suspension avant dosage des AGNE, à savoir des solutions de théophylline, d'isoprotérénol, de caféine, de Guarana titré à 12% en caféine et enfin une solution de composition selon l'invention.

A 540 µL de suspension d'adipocytes en milieu d'essai ont été ajoutés 60 µL de composé à tester, et les mélanges ont été incubés à 37°C, sous agitation, pendant 2 heures.

On s'est tenu à une concentration finale de théophylline dans le mélange testé de 1 mM, partant d'une solution stock de 10⁻² M en milieu d'essai.

De même, on s'est tenu à une concentration finale d'isoprotérénol dans le mélange testé de 1 µM, partant d'une solution stock de 10⁻² M en milieu d'essai.

Egalement, on s'est tenu à une concentration finale de caféine dans le mélange testé de 0,5 mM, partant d'une solution stock de 5 mM en milieu d'essai.

Le Guarana titré à 12% en caféine étant livré sous forme de poudre, une solution mère a été préparée à 20 mg/mL, soit 2% (p/v), en milieu d'essai. Partant de cette solution-mère, les tests ont été réalisés sur trois dilutions finales de la solution mère dans le mélange testé, 1%, 0,2% et 0,04%.

La composition selon l'invention étant une poudre, celle-ci a été diluée dans du DMSO d'une part et en milieu d'essai d'autre part. La solution-mère préparée au DMSO l'a été à 10 mg/mL, soit 1% (p/v), pour une concentration finale de composition selon l'invention en solution dans du DMSO dans le mélange testé de 0,01%.

La solution-mère préparée en milieu d'essai l'a été à 20 mg/mL, soit 2% (p/v), pour une concentration finale de composition selon l'invention en solution dans du milieu d'essai dans le mélange testé de 1% et 0,2 %.

Un témoin ne comprenant que les adipocytes en milieu d'essai a également été réalisé.

Au total, 10 dosages ont été réalisés à partir de 10 fractions différant par le composé ajouté ou non.

A l'issue du temps d'incubation, les AGNE ont été dosés dans des fractions de 30 µL de milieu, après décantation de 5 minutes. Le dosage a été réalisé conventionnellement par un kit de dosage NEFA-C.

Les données mesurées ont été traitées statistiquement. Des comparaisons intergroupes ont été réalisées par analyse de variance (ANOVA) à l'aide du test de comparaison multiple de Dunnett.

Les résultats de cette étude figurent en tableau 2 et figure 1, cette dernière consistant en une représentation graphique des résultats présentés en tableau 2.

La lipolyse basale, comme l'indique le témoin, était de 36 µM d'AGNE libérés en 2 heures.

La théophylline à 1 mM et l'isoprotérénol à 1 µM ont stimulé la lipolyse de façon significative (respectivement un facteur 10 par rapport au témoin, p<0.01). La caféine testée à 0.5 mM a nettement stimulé la libération d'AGNE (facteur 9.5 environ par rapport au témoin, p<0.01). Ainsi, les stimulations de lipolyse obtenues avec ces composés, qui sont des composés de référence aux effets connus, valident l'expérience de libération d'Acides Gras Non-Estérifiés (AGNE).

L'adjonction de composition selon l'invention solubilisée en milieu d'essai aqueux, testée à 1% et 0,2%, a pour résultat une nette stimulation de la libération d'AGNE (respectivement d'un facteur 7 et 6 environ par rapport au témoin). Ces stimulations s'avèrent dose-dépendantes. La composition selon l'invention solubilisée en DMSO et testée à 0,01% a stimulé la lipolyse avec une activité similaire à celle obtenue à 1% en milieu d'essai.

Enfin, la dilution de Guarana titré à 12% en caféine, testée à 1%, 0,2% et 0,04% a stimulé significativement et de façon dose-dépendante l'activité lipolytique (respectivement d'un facteur 5, 3 et 2,5 par rapport au témoin).

Par ailleurs, des contrôles d'absence d'interférence du produit avec le dosage des AGNE ont été systématiquement réalisés. Un contrôle négatif a été réalisé en mesurant la Densité Optique (DO) des fractions de composés aux concentrations finales utilisées (même milieu mais sans adipocytes). La présence de composé ne doit pas augmenter le signal de façon notable, c'est-à-dire que la présence de composé ne doit pas sensiblement modifier la DO de l'échantillon, ce qui peut être du à une forte DO du composé aux dilutions finales utilisées ou la présence d'une quantité non négligeable d'Acides Gras dans le composé à l'essai. Un contrôle positif a été réalisé en dosant une quantité connue d'AGNE (0,5 mM) en présence ou en l'absence du composé à l'essai. La présence du produit ne doit pas diminuer le signal de façon notable.

Dans les conditions expérimentales présentées, les composés n'ont pas présenté d'interférences avec le dosage des AGNE pour l'un ou l'autre des contrôles. Seule une légère interaction a été observée avec la composition selon l'invention diluée en DMSO, où le contrôle positif a montré une légère interaction (dosage des AGNE sous-évalué), mais cette interférence ne gêne pas l'interprétation des résultats.

En conclusion de cette expérience, il apparaît qu'à dose équivalente, dans les conditions de l'expérience, la composition selon l'invention présente une activité lipolytique supérieure à celle du Guarana, démontrant sans ambigüité la synergie des composants la constituant pour la stimulation de la lipolyse ex-vivo dans des adipocytes humains.

### EXPERIENCE 2

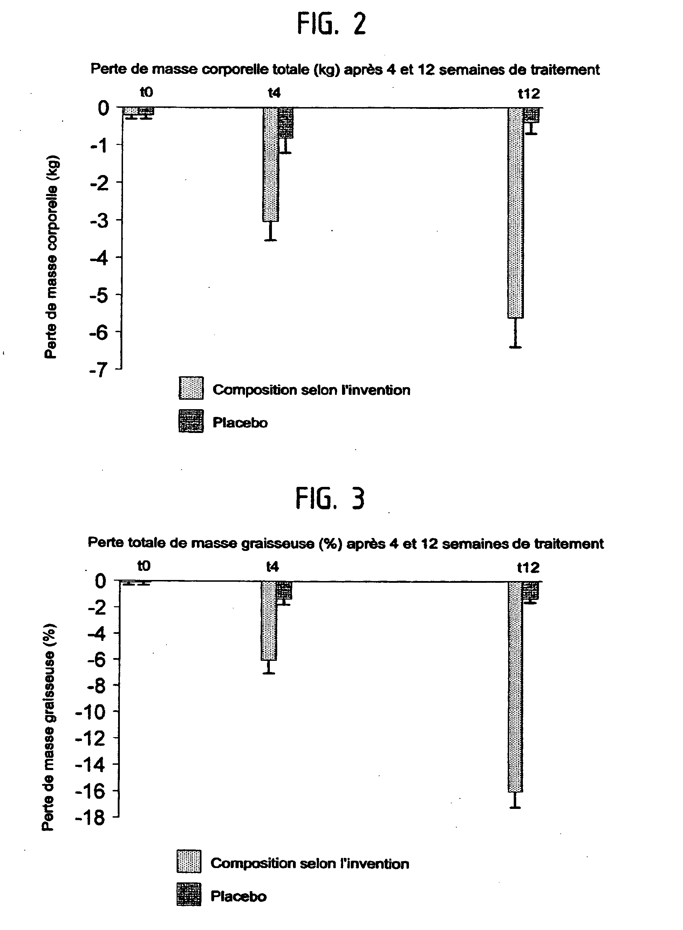

La seconde expérience consiste en une étude clinique dont les résultats figurent en tableau 3 à 6 et figures 2 et 3. Cette étude visait la détection des effets d'une administration de la composition selon l'invention sur des sujets en surpoids, comparativement à l'administration d'un placebo. Deux variables ont été contrôlées, à savoir la masse corporelle et le pourcentage de masse graisseuse.

En effet, ces variables sont des indicateurs macroscopiques de l'action de la composition selon l'invention sur l'activité de brûlage de graisses consécutive à la lipolyse et la perte de masse corporelle qui s'ensuit.

L'étude clinique a porté sur 20 sujets distribués en deux groupes :
- Un groupe nommé 'placebo' de 10 personnes (9 femmes et 1 homme), d'Indice de Masse Corporelle (IMC) entre 27 et 30 pour un IMC moyen de 28,5. L'âge des sujets de ce groupe est compris entre 22 et 55 ans et la masse corporelle moyenne constatée au début de l'étude était de 73 Kg.
- Un groupe nommé 'composition selon l'invention' de 10 personnes (7 femmes et 3 hommes), d'IMC moyen 28,1. Plus particulièrement, deux sous-groupes ont été distingués, le premier sous-groupe A (4 femmes) étant constitué de sujets considérés comme obèses, avec un IMC entre 29 et 33, le deuxième sous-groupe B (3 femmes, 3 hommes) étant constitué de sujets considérés en surpoids, avec un IMC entre 27 et 30. L'âge des sujets est compris entre 25 et 55 ans et la masse corporelle moyenne constatée au début de l'étude était de 70,5 Kg.

L'IMC est un indice très utilisé dans les études sur l'obésité, correspondant à la masse corporelle d'une personne en Kg, divisé par sa taille en mètres carrés (IMC = Kg/m²).

Malgré leur surpoids, les sujets de l'étude ont été choisis sans problèmes de santé (exclusion totale sur dysfonctionnements hépatiques, cardiovasculaires ou rénaux), hors grossesse, non-fumeurs, et non-consommateurs de médicaments ou tous autres suppléments alimentaires.

Les sujets ont été suivis pendant 12 semaines, pendant lesquelles la composition selon l'invention a été administrée au groupe "composition selon l'invention' sous forme de 4 capsules contenant chacune 350 mg de poudre de composition selon l'invention, à raison de 2 capsules le matin et 2 capsules durant le repas principal. Le groupe 'placebo' a reçu dans les mêmes formes un placebo.

Les sujets ont conservé leur quantité d'exercice physique habituel antérieur à l'étude ainsi que leurs habitudes alimentaires (entre 1500 et 2000 calories par jour).

Des mesures régulières du pourcentage de masse graisseuse des sujets par analyse d'impédance bioélectrique et de leur masse corporelle par pesée ont été réalisées durant ces 12 semaines.

Les tableaux 3 et 4 illustrent les données correspondant aux mesures de masses corporelle, respectivement chez les sujets du groupe 'placebo' et 'composition selon l'invention'.

Il apparaît clairement que, si la perte de masse corporelle est insignifiante, individuellement ou en moyenne, chez les sujets du groupe 'placebo', les sujets du groupe 'composition selon l'invention' ont vu leur masse corporelle baisser d'en moyenne 3 Kg à la semaine 4 et 5,6 Kg à la semaine 12.

La figure 2 illustre ces résultats en reprenant sous forme de graphique l'évolution de masse corporelle moyenne chez les deux groupes, notamment aux semaines 4 et 12.

Plus particulièrement, les sujets du sous-groupe A, ayant au départ un IMC important entre 29 et 33, ont montré la plus importante perte de masse corporelle à l'issue des 12 semaines d'étude.

Les tableaux 5 et 6 illustrent les données correspondant aux mesures de masses graisseuse, respectivement chez les sujets du groupe 'placebo' et 'composition selon l'invention'.

Il apparaît clairement que, si la perte de masse graisseuse est insignifiante, individuellement ou en moyenne, chez les sujets du groupe 'placebo', les sujets du groupe 'composition selon l'invention' ont vu leur masse graisseuse baisser d'en moyenne 5,9% à la semaine 4 et 15,6% à la semaine 12.

La figure 3 illustre ces résultats en reprenant sous forme de graphique l'évolution de la masse graisseuse moyenne chez les deux groupes, notamment aux semaines 4 et 12.

Plus particulièrement, les sujets du sous-groupe A, ayant au départ un IMC important entre 29 et 33, ont montré la plus importante perte de masse graisseuse à l'issue des 12 semaines d'étude.

Aucun effet secondaire majeur, tels que de la tachycardie ou une baisse de pression sanguine, n'a été observé chez l'un quelconque des sujets durant les 12 semaines de traitement avec 1,4 grammes quotidiens de composition selon l'invention.

Il ressort de cette étude clinique que la composition selon l'invention s'avère à la fois sûre et efficace quant à ses effets relatifs au brûlage de graisses et perte de masse corporelle.

Afin d'obtenir la composition selon l'invention, il a été procédé à une sélection de végétaux, notamment d'agrumes, dont la combinaison est particulièrement adaptée pour l'obtention de ladite composition. Le tableau 7 présente les espèces végétales essentielles nécessaires, selon un mode préférentiel de réalisation, où la composition est obtenue à partir d'un mélange d'oranges sanguines, d'oranges douces, d'oranges amères, de pamplemousses et, préférentiellement, de Guarana. Avantageusement, un pourcentage en poids fixant la représentation de chaque espèce végétale est respecté.

La normalisation en caféine pourra cependant, selon d'autres modes de réalisation, être obtenue à partir d'espèces végétales comme le Mate (*Ilex Paraguariensis* A.St.Hil) ou le Kola (*Cola Nitida* (Vent.) Schott & Endl.), selon toutes combinaisons entre ces espèces et avec le Guarana.

Les variétés d'orange sanguine retenues sont avantageusement de la liste suivante : *Citrus sinensis L. Osbeck CV 'Sanguinelli', Citrus sinensis L. Osbeck CV 'Tarocco', Citrus sinensis L. Osbeck CV 'Melitense', Citrus sinensis L. Osbeck CV 'St Michael' .*

L'origine naturelle de la composition selon l'invention et ses constituants expliquent le double mécanisme d'action qui lui permet de potentialiser l'action de la lipolyse dans les adipocytes. On rappellera que la composition selon l'invention est totalement exempte de synéphrine.

La structure polyphénolique de la majorité de ses constituants permet d'inhiber l'action de la cathéchol-o-méthyl transférase, tandis que son association unique de polyphénols et bases xanthiques permet d'inhiber l'action de la phosphodiestérase.

A titre de comparaison, l'action de la théophylline est connue pour s'exercer sur la cathéchol-o-méthyl transférase exclusivement et l'action de la caféine sur la phosphodiestérase.

Enfin, la composition selon l'invention est obtenue grâce à un procédé préférentiel, dont les étapes sont retranscrites ci-après. La figure 4 illustre par ailleurs à l'aide d'un diagramme les sous-étapes détaillées d'un mode d'exécution dudit procédé.

Ce procédé est totalement adapté à l'obtention d'une composition selon l'invention. Il est notamment basé sur deux points fondameataux : d'une part, il permet l'extraction sélective d'un ensemble de polyphénols (appartenant à plusieurs sous-groupes différents comme exposé dans le détail précédemment), ayant de façon synergique une activité de lipolyse, d'autre part il assure l'absence de synéphrine dans la composition finale, grâce à une étape de purification sélective sur colonne.

Plus particulièrement, concernant l'extraction sélective des polyphénols constitutifs de la composition selon l'invention, il a été nécessaire d'effectuer un mélange d'espèces végétales, notamment d'agrumes, pour l'obtention de la composition selon l'invention, les polyphénols recherchés n'étant pas tous présents dans une seule espèce végétale mais dans plusieurs. De plus, les différents polyphénols sont présents d'une manière dominante soit dans le jus, soit dans les écorces et/ou pépins des espèces végétales préalablement sélectionnées selon la règle précédente, d'où la nécessité d'effectuer une double extraction à partir du jus et à partir des écorces notamment.

Avantageusement, la composition selon l'invention peut encore être obtenue sous forme de poudre, hydrosoluble ou non.

Pour un meilleur exposé du procédé pour l'obtention de la composition selon l'invention, quatre étapes distinctes seront présentées :
- une première étape, appelée étape 1, consiste en l'extraction de polyphénols, notamment des anthocyanines et acides phénoliques, des jus des agrumes sélectionnés ;
- une deuxième étape, appelée étape 2, consiste en l'extraction de polyphénols, notamment des flavanones, des écorces et/ou pépins des agrumes sélectionnés ;
- une troisième étape, appelée étape 3, permet, suite au mélange des produits obtenus respectivement à l'issue des étapes 1 et 2, l'obtention d'une poudre de composition selon l'invention, ladite poudre étant insoluble dans l'eau ;
- une quatrième étape, appelée étape 4, permet, suite au mélange des produits obtenus respectivement à l'issue des étapes 1 et 2, l'obtention d'une poudre de composition selon l'invention, ladite poudre étant hydrosoluble ;

Il apparaît ainsi que les étapes 1 et 2 sont antérieures aux étapes 3 et 4, les étapes 1 et 2 pouvant cependant être conduites en parallèle. Les étapes 3 et 4 sont deux modes de réalisation alternatifs pour l'obtention d'une poudre de composition selon l'invention, l'étape 4 se distinguant de l'étape 3 par l'ajout d'étapes supplémentaires au procédé d'obtention d'une poudre selon l'étape 3.

Il est présenté, ci-dessous, un mode d'exécution dudit procédé selon l'invention, dans lequel les sous-étapes constitutives dudit procédé sont plus particulièrement détaillées. La figure 4 illustre par un diagramme la chronologie de ces sous-étapes en reprenant leurs références (les références figurant entre parenthèses dans la présente description).

### ETAPE 1

La première étape consiste en l'extraction de polyphénols des jus des agrumes sélectionnés. Plus particulièrement, les agrumes employés sont des oranges sanguines (*Citrus sinensis L. Osbeck*) et des oranges douces (*Citrus sinensis L. Osbeck*). Préférentiellement, des agrumes frais seront utilisés. Il reste possible d'utiliser des agrumes séchés ou congelés.

Les anthocyanines appartiennent à la famille des polyphénols et sont caractérisés par la présence de deux cycles benzéniques qui entourent un cycle pyrylium d'un enchaînement de carbones C3-C6-C3. Dans l'orange sanguine *(Citrus sinensis L. Osbeck)* les anthocyanines se trouvent sous forme de sucres (monosaccharides tels que glucose). La glycosilation se fait en position 3 des anthocyanidines (forme aglycone). L'anthocyanine dominante dans l'orange sanguine (*Citrus sinensis L. Osbeck*) est la Cyanidine-3-glucoside. Les acides phénoliques sont également présents dans les jus des agrumes.

Ainsi l'étape 1 permet plus particulièrement l'extraction des anthocyanines et acides phénoliques de ces agrumes.

La vitamine C est également présente dans le jus des agrumes, et extraite dans le même temps.

Les agrumes sélectionnés sont pressés à froid (1a), individuellement ou combinés en différents groupes.

Le pH des jus obtenus du pressage est immédiatement amené (1b) à une valeur comprise entre 1,5 et 2 par ajout d'acide chlorhydrique (HCl) 0,1% 12 M ou d'acide formique 10%. Une faible valeur de pH est nécessaire pour maintenir les anthocyanines essentiellement sous forme de cation flavylium (forme la plus stable). Une étude préalable a montré que 97% des anthocyanines se trouvent sous forme de cation flavylium à pH compris entre 1,5 et 2, et uniquement 55% quand la valeur de pH est supérieure à 2,5.

Les jus sont ensuite centrifugés (1c) pendant 30 minutes à 3 heures à 4000 tours/min, et la phase liquide recueillie en vue d'une purification par séparation (1d) sur colonne.

Une double élution avec des solvants différents est conventionnellement réalisée afin d'éliminer notamment la synéphrine (la synéphrine étant totalement éliminée dans le cadre de la composition selon l'invention). Les anthocyanines et acides phénoliques ont la capacité de se fixer réversiblement sur un grand nombre de colonnes absorbantes, permettant ainsi d'éliminer notamment la synéphrine. La colonne utilisée peut avantageusement être composée d'un mélange de polyvinylpyrrolidone (pvp) et de gel (Sephadex G25) dans un rapport respectif de 70:30 à 95:5. On pourra bien entendu choisir d'autres types de colonnes, même si ce mélange de support s'est avéré le plus efficace pour une bonne séparation, par sa bonne rétention des anthocyanines et acides phénoliques quand le jus passe à travers la colonne.

Un fractionnement en deux parties est obtenu en fonction des solvants d'élution utilisés. Une première fraction est obtenue après élution de la colonne avec de l'eau, celle-ci entraînant les sucres, les acides et les polysaccharides solubles dans la première fraction. Une seconde fraction est obtenue après élution avec un mélange de solvants Ethanol/Eau/Acide formique (respectivement dans des proportions 30:68:2 à 83:15:2), permettant d'obtenir principalement les anthocyanines-3-monoglycosides et acides phénoliques dans la seconde fraction.

Le contrôle de ces deux fractions par analyse HPLC a montré l'absence de substances interférentes comme la synéphrine ou l'octopamine.

Les fractions résultantes sont ensuite mélangées (1e) et le mélange concentré (1f) sous vide, ce qui permet d'éliminer la totalité des solvants utilisés et une grande partie de l'eau pour obtenir un concentré que nous appellerons concentré 1.

Si 50% de la masse des agrumes est constitué par leur jus, il résulte de cette seule exploitation une quantité importante de sous-produits (principalement des écorces et des pépins). Ces sous-produits seront ainsi utilisés dans l'étape 2.

### ETAPE 2

La deuxième étape consiste en l'extraction de polyphénols des sous-produits des agrumes sélectionnés. Plus particulièrement, les agrumes employés sont des oranges sanguines (*Citrus sinensis L. Osbeck*)*,* des pamplemousses (*Citrus paradisi* Macfad.), des oranges douces (*Citrus sinensis L. Osbeck)* et des oranges amères (*Citrus aurantium L.*).

Préférentiellement, on choisira des sous-produits d'agrumes frais, mais ceux-ci pourront également être séchés ou congelés.

Ainsi l'étape 2 permet plus particulièrement l'extraction des flavanones de ces agrumes.

Un mélange de sous-produits d'orange sanguine (*Citrus sinensis L. Osbeck*), de pamplemousse (*Citrus paradisi* Macfad.), d' orange douce *(Citrus sinensis* L. *Osbeck*) et d' orange amère (*Citrus* aurantium L.) est placé dans un extracteur (2a) pendant 1 à 12 heures à 40-70° Celsius en présence de solvants, par exemple un mélange d'Ethanol/Méthanol/Eau dans des proportions respectives 40:10:50.

Le mélange peut ensuite rester au repos (2b) dans l'extracteur pendant 4 à 6 heures.

Le contenu de l'extracteur est ensuite centrifugé (2c) pendant 30 minutes à 3 heures à 4000 tours/min. La phase solide est séparée et la phase liquide est introduite dans une colonne de purification (2d). Celle-ci est avantageusement, mais non exclusivement une colonne de résine. Les flavanones sont obtenues après élution de la colonne avec un mélange de solvants Ethanol/Méthanol/Eau dans des proportions respectives 65:5:30.

L'extrait final résultant, au besoin après mélange (2e, étape facultative) de différents extraits issus des précédentes étapes, est ensuite concentré (2f) sous vide pour obtenir un concentré que nous appelleront concentré 2.

### ETAPE 3

La troisième étape consiste à mélanger (3a) les deux concentrés 1 et 2 en vue de l'obtention d'une poudre. Ainsi, les extraits 1 et 2 mélangés sont séchés par atomisation (3b) sur un support maltodextrine. Le produit obtenu est normalisé en caféine (3c), par mélange avec un extrait de Guarana (*Paullinia cupana Kunth*) titré 12% en caféine naturelle, selon les rapports Composition selon l'invention/extrait de Guarana de 80:20 à 95:5. La teneur finale en caféine brute varie alors entre 0,1 et 4% de la composition selon l'invention. D'autres sources conventionnelles de caféine pourront être utilisées pour atteindre cet objectif, comme par exemple l'emploi de maté ou de kola. Le produit fini est tamisé (3d) pour obtenir une poudre de composition selon l'invention insoluble dans l'eau, de granulométrie entre 40 et 100 mesh.

### ETAPE 4

La quatrième étape consiste à mélanger (4a) les deux concentrés 1 et 2 en vue de l'obtention d'une poudre hydrosoluble. Ainsi, les extraits 1 et 2 mélangés sont mis en solution avec de l'eau (4b), dans un rapport d'une unité de mélange des deux concentrés pour 10 unités d'eau, et amenés à 20° Celsius pendant 1 à 5 heures sous agitation (4c) et en atmosphère inerte.

On procède ensuite à la séparation des particules insolubles par décantation (4d) pendant 1 à 3 heures. On prélève ensuite la fraction de surface. Le dépôt insoluble est repris à l'eau dans une nouvelle séparation identique à celle précédemment décrite (4b à 4d).

Les fractions liquides récupérées sont alors filtrées (4e) sur papier filtre. Le ratio final des deux fractions Insoluble/Soluble est respectivement entre 15 à 30 % de fraction insoluble pour 85 à 70 % de fraction soluble.

Les fractions hydrosolubles sont ensuite soumises au même traitement que décrit en étape 3, c'est-à-dire concentrées (4f) sous vide et séchées par atomisation (4g) sur de la maltodextrine.

Le produit obtenu est normalisé en caféine (4h), par mélange mélangé avec un extrait de Guarana (*Paullinia cupana Kunth*) titré 12% en caféine naturelle, selon les rapports Composition selon l'invention/Guarana de 80:20 à 95:5. La teneur finale en caféine brute varie alors entre 0,1 et 4% dans la composition selon l'invention. D'autres sources conventionnelles de caféine pourront être utilisées pour atteindre cet objectif, comme par exemple l'emploi de maté ou de kola. Le produit fini est tamisé (4i) pour obtenir une poudre de composition selon l'invention soluble dans l'eau, de granulométrie entre 40 et 100 mesh.

Au travers du procédé selon l'invention, il est ainsi possible d'obtenir une composition répondant avantageusement au problème posé, c'est-à-dire une composition à activité lypolitique permettant d'encourager la perte de masse corporelle et le raffermissement corporel assisté par l'exercice musculaire, dans la mesure où la lipolyse conduit au brûlage des graisses et peut ainsi permettre d'augmenter les performances physiques. La composition obtenue est en outre totalement exempte de synéphrine.

La poudre obtenue à l'issue du procédé selon l'invention peut être hydrosoluble.

L'invention concerne encore l'utilisation de la composition obtenue comme complément alimentaire ou pour enrichir des aliments.

Son emploi comme complément alimentaire sera facilité sous sa forme de poudre insoluble dans l'eau. Dans ce contexte et de manière non exhaustive, la poudre insoluble dans l'eau obtenue peut être employée, seule ou avec des adjuvants supplémentaires, sous forme galénique notamment de poudre encapsulée, par exemple en gélule, de poudre compressée en comprimés ou granulés de toutes formes.

Son emploi pour enrichir les aliments sera facilité sous sa forme de poudre hydrosoluble. La poudre hydrosoluble peut en effet avantageusement être employée, seule ou avec des adjuvants supplémentaires, par exemple en étant dissoute dans un liquide alimentaire, et ainsi incorporée à des boissons, ou bien dans des produits lactés tels des yaourts. Elle peut encore être, de manière non exhaustive, incorporée dans des biscuits ou des confiseries, ou présentée sous forme de sachets hydrosolubles.

De manière non exhaustive, les boissons peuvent par exemple être des jus de fruits et/ou légumes.

L'invention concerne encore l'utilisation de la composition obtenue pour son administration diététique.

Finalement, on rappellera que l'administration de la composition selon l'invention permet avantageusement de favoriser le brûlage des graisses ou la perte de masse corporelle. Elle est particulièrement efficace chez les individus ayant un IMC de type obèse, c'est-à-dire supérieur à 30.

La posologie étudiée correspond à la posologie préférentielle, qui est d'environ 1,4 grammes par jour de composition. Cette posologie peut naturellement être adaptée selon la réponse physiologique au traitement, et varier entre 1,0 et 2,0 grammes par jour et par individu.

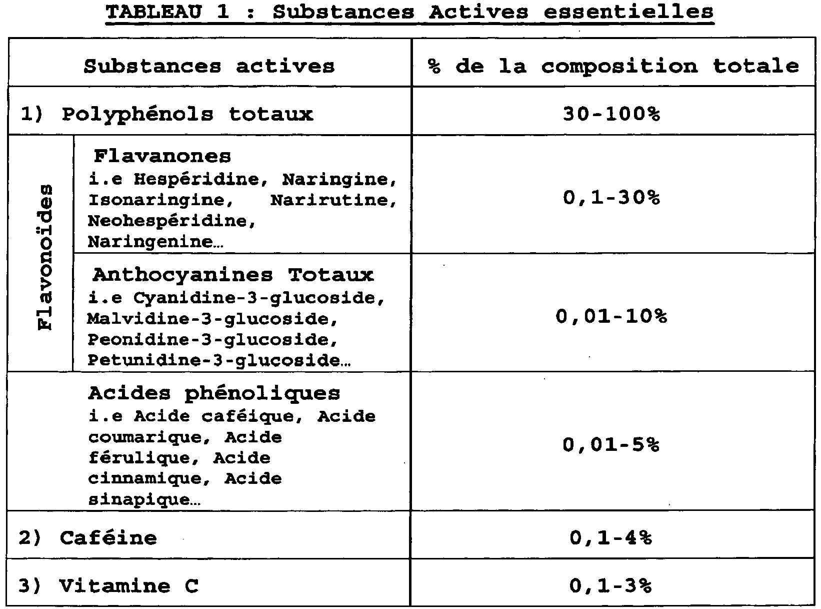

**TABLEAU 2 EXPERIENCE 1 - Dosage des AGNE libérés**

| Traitement | Concentration | AGNE (µM) | sd | n | % du témoin | p |
|---|---|---|---|---|---|---|
| Témoin non traité | - | 36 | 10 | 3 | 100 | - |
| Théophylline | 1,0 mM | 381 | 9 | 3 | 1057 | p<0,01 |
| Isoprotérénol | 1,0 µM | 377 | 11 | 3 | 1048 | p<0.01 |
| Caféine | 0,5 mM | 339 | 4 | 3 | 941 | p<0,01 |
| Composition selon l'invention en milieu d'essai | 1,0% | 244 | 17 | 3 | 677 | p<0,01 |
| | 0,2% | 213 | 13 | 3 | 592 | p<0,01 |
| Composition selon l'invention en DMSO | 0,01% | 231 | 6 | 3 | 643 | p<0,01 |
| | 1,0% | 178 | 15 | 3 | 494 | p<0,01 |
| Guarana 12% | 0,2% | 101 | 11 | 3 | 281 | p<0,01 |
| | 0,4% | 90 | 17 | 3 | 249 | p<0,01 |

| | | | | | | |
|---|---|---|---|---|---|---|
| sd : écart-type n : nombre d'observations p : résultat de l'analyse de variance | | | | | | |

**TABLEAU 3 : EXPERIENCE 2 - Evolution de la masse corporelle après administration d'un placebo**

| | Age | Sexe | IMC | Masse corporelle t0 (Kg) | Perte de masse corporelle t4 (Kg) | Perte de masse corporelle t12 (Kg) |
|---|---|---|---|---|---|---|
| Sujet 1 | 22 | F | 28,8 | 71,9 | -1,4 | -1,4 |
| Sujet 2 | 33 | F | 28,5 | 70,2 | -0,8 | -1,3 |
| Sujet 3 | 29 | F | 28,2 | 71,4 | -0,5 | -0,4 |
| Sujet 4 | 25 | F | 29,3 | 67,7 | -0,9 | +2,9 |
| Sujet 5 | 55 | F | 28,2 | 74,1 | -0,2 | -0,2 |
| Sujet 6 | 45 | F | 26,9 | 68, 8 | -0,7 | -0,8 |
| Sujet 7 | 28 | F | 29,1 | 78,4 | -1,2 | -1,0 |
| Sujet 8 | 47 | M | 28,2 | 84,6 | -0,9 | -0,7 |
| Sujet 9 | 25 | F | 28,4 | 72,8 | -0,8 | -0,8 |
| Sujet 10 | 29 | F | 29,4 | 70,7 | -0,5 | -0,7 |
| Moyenne | 33,1 | | 28,5 | 73 | -0,8 | -0,4 |

**TABLEAU 4 EXPERIENCE 2 - Evolution du masse corporelle après administration de composition selon l'invention**

| | Age | Sexe | IMC | Masse corporelle t0 (Kg) | Perte de masse corporelle t4 (Kg) | Perte de masse corporelle t12 (Kg) |
|---|---|---|---|---|---|---|
| Sujet 1 | 27 | F | 26,6 | 63,8 | -2,4 | -3,6 |
| Sujet 2 | 33 | M | 27,0 | 69,1 | -1,4 | -3,0 |
| Sujet 3 | 35 | F | 25,1 | 60,2 | -1,7 | -3,2 |
| Sujet 4* | 25 | F | 28,9 | 73,9 | -3,5 | -7,5 |
| Sujet 5 | 55 | M | 26,8 | 73,0 | -3,0 | -4,1 |
| Sujet 6* | 47 | F | 31,8 | 80,5 | -5,3 | -8,9 |
| Sujet 7* | 22 | F | 32,7 | 78,6 | -3,2 | -8,8 |
| Sujet 8 | 39 | M | 25,3 | 72,2 | -4,1 | -5,1 |
| Sujet 9* | 41 | F | 29,3 | 68,7 | -3,7 | -8,5 |
| Sujet 10 | 37 | F | 27,5 | 65,3 | -2,0 | -3,4 |
| Moyenne | 36,1 | | 28,1 | 70,5 | -3,0 | -5,6 |

| | | | | | | |
|---|---|---|---|---|---|---|
| * Sujets appartenant au groupe d'IMC compris entre 29 et 33 | | | | | | |

**TABLEAU 5 EXPERIENCE 2 - Evolution de la masse graisseuse après administration d'un placebo**

| | Age | Sexe | IMC | Masse graisseuse t0 (%) | Evolution de la masse graisseuse t4 (%) | Evolution de la masse graisseuse t12 (%) |
|---|---|---|---|---|---|---|
| Sujet 1 | 22 | F | 28,8 | 31,8 | -0,3 | -0,3 |
| Sujet 2 | 33 | F | 28,5 | 33,9 | -0,4 | -0,5 |
| Sujet 3 | 29 | F | 28,2 | 32,5 | -0,4 | -0,5 |
| Sujet 4 | 25 | F | 29,3 | 31,8 | -0,9 | +0,1 |
| Sujet 5 | 55 | F | 28,2 | 32,5 | -0,4 | -0,1 |
| Sujet 6 | 45 | F | 26,9 | 29,8 | -0,5 | -0,3 |
| Sujet 7 | 28 | F | 29,1 | 31,5 | -0,6 | -0,3 |
| Sujet 8 | 47 | M | 28,2 | 32,7 | -0,2 | -0,5 |
| Sujet 9 | 25 | F | 28,4 | 31,7 | -0,2 | -1,8 |
| Sujet 10 | 29 | F | 29,4 | 32,7 | -0,5 | +0 |
| Moyenne | 33,1 | | 28,5 | 32,1 | -0,4 | -0,4 |
| Pourcentage de diminution de la masse graisseuse | | | | | 1,25% | 1,25% |

**TABLEAU 6 : EXPERIENCE 2 - Evolution de la masse graisseuse après administration de composition selon l'invention**

| | Age | Sexe | IMC | Masse graisseuse t0 (%) | Evolution de la masse graisseuse t4 (%) | Evolution de la masse graisseuse t12 (%) |
|---|---|---|---|---|---|---|
| Sujet 1 | 27 | F | 26,6 | 28,7 | -0,8 | -2,0 |
| Sujet 2 | 33 | M | 27,0 | 29,2 | -0,6 | -2,4 |
| Sujet 3 | 35 | F | 25,1 | 30,1 | -0,6 | -2,0 |
| Sujet 4* | 25 | F | 28,9 | 33,1 | -3,4 | -10,2 |
| Sujet 5 | 55 | M | 26,8 | 28,7 | -1,2 | -2,2 |
| Sujet 6* | 47 | F | 31,8 | 32,9 | -3,1 | -8,7 |
| Sujet 7* | 22 | F | 32,7 | 32,2 | -2,9 | -6,8 |
| Sujet 8 | 39 | M | 25,3 | 29,2 | -0,6 | -2,4 |
| Sujet 9* | 41 | F | 29,3 | 33,7 | -3,5 | -9,8 |
| Sujet 10 | 37 | F | 27,5 | 29,5 | -0,8 | -1,8 |
| Moyenne | 36,1 | | 28,1 | 30,7 | -1,8 | -4,8 |
| Pourcentage de diminution de la masse graisseuse | | | | | 5,9% | 15,6% |

| | | | | | | |
|---|---|---|---|---|---|---|
| * Sujets appartenant au groupe d'IMC compris entre 29 et 33 | | | | | | |

**TABLEAU 7 : Espèces végétales essentielles**

| Espèces végétales de la composition | Nom botanique | % de la composition |
|---|---|---|
| Agrumes | | |
| Orange sanguine | *Citrus sinensis L. Osbeck* | 5-30% |
| Pamplemousse | *Citrus paradisi* Macfad. | 5-30% |
| Orange douce Orange amère | *Citrus sinensis L. Osbeck Citrus aurantium L.* | 5-25% 1-10% |
| Autres végétaux | | |
| Guarana | *Paullinia cupana Kunth* | 5-20% |

## Revendications

1. Composition à activité lipolytique comprenant des polyphénols, notamment sous forme de flavonoïdes, en particulier des flavanones et des anthocyanines, et d'acides phénoliques, et de la caféine, lesdits polyphénols étant représentés par au moins 0,1 à 30% de flavanones, 0,01 à 10% d'anthocyanines et 0,01 à 5% d'acides phénoliques, les pourcentages se référant à la composition totale et le total de polyphénols présents atteignant au moins 30% de la composition totale, **caractérisée en ce qu'**elle est obtenue à partir d'un mélange d'espèces végétales comportant au moins 5 à 30% en poids d'oranges sanguines (*Citrus sinensis L. Osbeck*), 5 à 30% en poids de pamplemousses (*Citrus paradisi* Macfad.), 5 à 25% en poids d'oranges douces (*Citrus sinensis L*. *Osbeck*), 1 à 10% en poids d'oranges amères (*Citrus aurantium* L.), 5 à 20% en poids de Guarana (*Paullinia cupana Kunth*) et **en ce que** ladite composition est exempte de synéphrine.

2. Composition à activité lipolytique selon la revendication 1, **caractérisée en ce qu'**elle comprend en plus de la vitamine C.

3. Composition à activité lipolytique selon l'une des revendications précédentes, **caractérisée en ce qu'**elle comprend entre 60 et 90% de polyphénols.

4. Composition à activité lipolytique selon l'une des revendications précédentes, **caractérisée en ce qu'**elle comprend 0,1 à 4% de caféine.

5. Composition à activité lipolytique selon l'une des revendications précédentes, **caractérisée en ce qu'**elle comprend 0,1 à 3% de vitamine C.

6. Composition à activité lipolytique selon l'une des revendications précédentes, **caractérisée en ce qu'**elle se présente, seule ou avec des adjuvants supplémentaires, sous forme d'une poudre ou d'une poudre hydrosoluble.

7. Composition à activité lipolytique selon l'une des revendications précédentes, **caractérisée en ce qu'**elle se présente, seule ou avec des adjuvants supplémentaires, sous forme d'une poudre encapsulée, notamment sous forme de gélules, d'une poudre compressée, notamment sous forme de comprimés ou granulés de toutes formes, ou sous forme d'une poudre ensachée.

8. Procédé pour l'obtention d'une composition à activité lipolytique selon l'une des revendications 1 à 7, **caractérisé par le fait qu'**il comporte, à partir d'un mélange d'espèces végétales comportant au moins 5 à 30% en poids d'oranges sanguines *(Citrus sinensis L. Osbeck),* 5 à 30% en poids de pamplemousses *(Citrus paradisi* Macfad.), 5 à 25% en poids d'oranges douces *(Citrus sinensis L. Osbeck),* 1 à 10% en poids d'oranges amères *(Citrus aurantium L.*), et 5 à 20% en poids de Guarana (*Paullinia cupana Kunth*) au moins :
- une étape d'extraction de polyphénols de jus d'orange sanguine (*Citrus sinensis L. Osbeck*) et d'orange douce (*Citrus sinensis L. Osbeck*),
- une étape d'extraction de polyphénols de sous-produits d'orange sanguine (*Citrus sinensis L. Osbeck*), de pamplemousse (*Citrus paradisi* Macfad.), d'orange douce *(Citrus sinensis L. Osbeck)* et d'orange amère *(Citrus aurantium L.*),
- une étape de mélange des extraits obtenus des étapes d'extraction précédentes.
- suite à l'étape de mélange une étape de séchage du mélange par atomisation sur un support maltodextrine,
- suite à l'étape de séchage, une étape de normalisation en caféine au produit résultant de l'étape de séchage,
- puis enfin une étape de tamisage du produit résultant afin d'obtenir une poudre.

9. Procédé pour l'obtention d'une composition à activité lipolytique selon la revendication 8, **caractérisé par le fait que** l'étape de normalisation en caféine est réalisée par l'ajout d'un extrait de Guarana (*Paullinia cupana Kunth*), et/ou de Mate (*Ilex Paraguariensis* A.St.Hil), et/ou de Kola (*Cola Nitida* (Vent.) Schott & Endl.), au produit résultant de l'étape de séchage.

10. Procédé pour l'obtention d'une composition à activité lipolytique selon l'une des revendications 8 ou 9, **caractérisé par le fait que** l'on procède à l'extraction des anthocyanines et acides phénoliques de jus d'orange sanguine (*Citrus sinensis L. Osbeck*) et d'orange douce (*Citrus sinensis L.Osbeck*) et, à l'extraction de flavanones de sous-produits d'orange sanguine (*Citrus sinensis L. Osbeck*), de pamplemousse (*Citrus paradisi* Macfad.), d'orange douce (*Citrus sinensis L. Osbeck*) et d'orange amère (*Citrus aurantium L.*).

11. Procédé pour l'obtention d'une composition à activité lipolytique selon l'une des revendications 8 à 10, **caractérisé par le fait que**, préalablement à l'étape de séchage, on procède à une étape d'élimination des particules insolubles dans l'eau du mélange.

12. Utilisation de la composition à activité lipolytique selon les revendications 1 à 7 comme complément alimentaire.

13. Utilisation de la composition à activité lipolytique selon les revendications 1 à 7 pour enrichir les aliments, notamment les boissons telles que les jus de fruits et/ou légumes ainsi que les produits lactés.

14. Utilisation non thérapeutique de la composition à activité lipolytique selon les revendications 1 à 7, pour son administration en vue de favoriser le brûlage des graisses.

15. Utilisation non thérapeutique de la composition à activité lipolytique selon les revendications 1 à 7, pour son administration en vue de favoriser la perte de masse corporelle.

16. Utilisation non thérapeutique de la composition à activité lipolytique selon les revendications 1 à 7 pour son administration à activité lipolytique suivant une posologie entre 1,0 et 2,0 grammes, préférentiellement 1,4 grammes, de composition par jour.

## Patentansprüche

1. Zusammensetzung mit lipolytischer Aktivität, umfassend Polyphenole, nämlich in der Form von Flavonoiden, insbesondere Flavanonen und Anthocyanen, und Phenolsäuren und Koffein, wobei die Polyphenole aus mindestens 0,1 bis 30% Flavanonen, 0,01 bis 10% Anthocyaninen und 0,01 bis 5% Phenolsäuren bestehen, die Prozentangaben auf die Gesamtzusammensetzung bezogen sind, und wobei die Gesamtmenge der vorhandenen Polyphenole mindestens 30% der gesamten Zusammensetzung erreicht, **dadurch gekennzeichnet, dass** sie ab einer Mischung von Pflanzenarten erhalten wird, die mindestens 5 bis 30 Gew-% Blutorangen (*Citrus sinensis L. Osbeck*), 5 bis 30 Gew-% Grapefruit *(Citrus paradisi Macfad.),* 5 bis 25 Gew-% Süßorangen (*Citrus sinensis L. Osbeck*), 1 bis 10 Gew-% Bitterorangen (*Citrus aurantium L.*), 5 bis 20 Gew-% Guarana (*Paullinia cupana Kunth*) umfasst, und daß die Zusammensetzung synephrinfrei ist.

2. Zusammensetzung mit lipolytischer Aktivität nach Anspruch 1, **dadurch gekennzeichnet, daß** es weiterhin Vitasin C enthält.

3. Zusammensetzung mit lipolytischer Aktivität nach einem der vorgehenden Ansprüche, **dadurch gekennzeichnet, dass** sie zwischen 60 und 90% Polyphenole enthält.

4. Zusammensetzung mit lipolytischer Aktivität nach einem der vorgehenden Ansprüche, **dadurch gekennzeichnet, daß** sie 0,1 bis 4% Koffein enthält.

5. Zusammensetzung mit lipolytischr Aktivität nach einem der vorgehenden Ansprüche, **dadurch gekennzeichnet, daß** sie 0,1 bis 3% Vitamin C umfasst.

6. Zusammensetzung mit lipolytischer Aktivität nach einem der vorgehenden Ansprüche, **dadurch gekennzeichnet, daß** sie, allein oder mit weiteren Hilfsstoffen, in der Form von Pulver oder wasserlöslichem Pulver ist.

7. Zusammensetzung mit lipolytischer Aktivität nach einem der vorgehenden Ansprüche, **dadurch gekennzeichnet, daß** sie, allein oder mit weiteren Hilfsstoffen, in der Form von eine eingekapselten Pulver, nämlich in der Form von Kapseln, komprimiertem Pulver, nämlich in der Form von Tabletten oder Granulaten jeder Form, oder von Pulver in Beutelchen ist.

8. Verfahren zum Erhalten einer Zusammensetzung mit lipolytischer Aktivität nach einem der Ansprüche 1 bis 7, **dadurch gekennzeichnet, dass** es ab einer Mischung von Pflanzenarten, die mindestens 5 bis 30 Gew-% Blutorangen (*Citrus sinensis L. Osbeck*), 5 bis 30 Gew-% Grapefruit (*Citrus paradisi Macfad.*), 5 bis 25 Gew-% Süßorangen (*Citrus sinensis L. Osbeck*), 1 bis 10 Gew-% Bitterorangen (*Citrus aurantium L.*), 5 bis 20 Gew-% Guarana (*Paullinia cupana Kunth*) umfasst, mindestens nachstehende Schritte umfasst:
- einen Schritt des Extrahierens von Polyphenolen aus Saft von Blutorangen (*Citrus sinensis L. Osbeck*) und Süßorangen (*Citrus sinensis L. Osbeck*),
- einen Schritt des Extrahierens von Polyphenolen aus Nebenprodukten von Blutorangen (*Citrus sinensis L. Osbeck*), Grapefruit (*Citrus paradisi Macfad.*), Süßorangen (*Citrus sinensis L. Osbeck*) und Bitterorangen (*Citrus aurantium L*.),
- eineen Schritt des Mischens der in den vorgehenden Extraktionsschritten erhaltenen Extrakte,
- nach dem Schritt des Mischens einen Schritt der Sprühtrocknung der Mischung auf einem Maltodextrinträger,
- nach dem Trocknungsschritt einen Schritt der Normalisierung in Koffein des sich aus dem Trocknungsschritt ergebenden Produkts,
- dann schließlich einen Schritt des Siebens des sich ergebenden Produkts, um ein Pulver zu erhalten.

9. Verfahren zum Erhalten einer Zusammensetzung mit lipolytischer Aktivität nach Anspruch 8, **dadurch gekennzeichnet, dass** der Schritt der Normalisierung in Koffein durch Zugabe eines Extraktes von Guarana (*Paullinia cupana Kunth*) und/oder Mate (*Ilex Paraguariensis* A.St.Hil), und/oder Kola (*Cola nitida* (Vent). Schott & Endl.) zu dem sich aus dem Trocknungsschritt ergebenden Produkt durchgeführt wird.

10. Verfahren zum Erhalten einer Zusammensetzung mit lipolytischer Aktivität nach einem der Ansprüche 8 oder 9, **dadurch gekennzeichnet, daß** das Extrahieren von Anthocyanen und phenolischen Säuren aus Saft von Blutorangen (*Citrus sinensis L. Osbeck*) und Süßorangen (*Citrus sinensis L. Osbeck)* und das Extrahieren von Flavanonen aus Nebenprodukten von Blutorangen (*Citrus sinensis L. Osbeck*), Grapefruit (*Citrus paradisi Macfad.*) Süßorangen (*Citrus sinensis L. Osbeck)* und Bitterorangen (*Citrus aurantium L.*) vorgenommen werden.

11. Verfahren zum Erhalten einer Zusammensetzung mit lipolytischer Aktivität nach einem der Ansprüche 8 bis 10, **dadurch gekennzeichnet, dass** vor dem Trocknungsschritt ein Schritt des Entfernens waaserunlöslicher Partikel aus dem Gemisch vorgenommen wird.

12. Verwendung der Zusammensetzung mit lipolytischer Aktivität nach einem der Ansprüche 1 bis 7 als Nahrungsergänzungsmittel.

13. Verwendung der Zusammensetzung mit lipolytischer Aktivität nach einem der Ansprüche 1 bis 7 zur Anreicherung von Lebensmitteln, nämlich Getränken, wie Frucht- und/oder Gemüsesäften sowie Milchprodukten.

14. Nicht-therapeutische Verwendung der Zusammensetzung mit lipolytischer Aktivität nach einem der Ansprüche 1 bis 7 zur Verabreichung zwecks der Förderung von Fettverbrennung.

15. Nicht-therapeutische Verwendung der Zusammensetzung mit lipolytischer Aktivität nach einem der Ansprüche 1- bis 7 zur Verabreichung zwecks der Förderung von Körpergewichtsverlust.

16. Nicht-therapeutische Verwendung der Zusammensetzung mit lipolytischer Aktivität nach einem der Ansprüche 1 bis 7 für die Verabreichung mit lipolytischer Aktivität in einer Dosis zwischen 1,0 und 2,0 Gramm, vorzugsweise 1,4 Gramm der Zusammensetzung pro Tag.

## Claims

1. A composition with lipolytic activity, comprising polyphenols, namely in the form of flavonoids, in particular flavanones and anthocyanins, and phenolic acids, and caffeine, said polyphenols being represented by at least 0.1 to 30% flavanones, 0.01 to 10% anthocyanins and 0.01 to 5% phenolic acids, the percentages referring to the total composition and the total of polyphenols present amounting to at least 30% of the total composition, wherein the composition is obtained from a mixture of plant species comprising at least 5 to 30 weight % of blood oranges (*Citrus sinensis L. Osbeck*), 5 to 30 weight % of grapefruit (*Citrus parade Macfad.*), 5 to 25 % weight of sweet oranges (*Citrus sinensis L. Osbeck*), 1 to 10 weight % of bitter oranges (*Citrus aurantium L.*), 5 to 20 weight % of Guarana (*Paullinia cupana Kunth*), and wherein said composition is synephrine-free.

2. A composition with lipolytic activity according to claim 1, wherein it further comprises vitamin C.

3. A composition with lipolytic activity according to one of the preceding claims, wherein it comprises from 60 to 90% polyphenols.

4. A composition with lipolytic activity according to one of the preceding claims, wherein it comprises from 0.1 to 4% caffeine.

5. A composition with lipolytic activity according to one of the preceding claims, wherein it comprises from 0.1 to 3% vitamin C.

6. A composition with lipolytic activity according to one of the preceding claims, wherein it is, alone or with additional adjuvants, in the form of a powder or a water-soluble powder.

7. A composition with lipolytic activity according to one of the preceding claims, wherein it is present, alone or with additional adjuvants, in the form of an encapsulated powder, namely in the form of capsules, of a compressed powder, namely in the form of tablets or granulates of any form, or in the for of bagged powder.

8. A method for obtaining a composition with lipolytic activity according to one of claims 1 to 7, wherein the method comprises, starting from a mixture of plant species comprising at least 5 to 30 weight % of blood oranges (*Citrus sinensis L. Osbeck*), 5 to 30 weight % of grapefruit (*Citrus paradisi Macfad.*), 5 to 25 % weight of sweet oranges (*Citrus sinensis L. Osbeck),* 1 to 10 weight % of bitter oranges (*Citrus aurantium L.*), 5 to 20 weight % of Guarana (*Paullinia cupana Kunth*), at least:
- a step of extracting polyphenols from blood orange (*Citrus sinensis L. Osbeck*) and sweet orange (*Citrus sinensis L. Osbeck*) juice,
- a step of extracting polyphenols from blood orange (*Citrus sinensis L. Osbeck*), grapefruit (*Citrus paradisi Macfad.*), sweet orange (*Citrus sinensis L. Osbeck*) and bitter orange (*Citrus aurantium L.*) by-products
- a step of mixing the extracts obtained in the preceding extraction steps.
- following the mixing step, a step of spray drying the mixture on a maltodextrin carrier,
- following the drying step, a step of normalizing the caffeine in the product resulting from the drying step,
- then finally a step of sifting the resulting product, in order to obtain a powder.

9. A method for obtaining a composition with lipolytic activity according to claim 8, wherein the step of normalizing caffeine is carried out by adding an extract of Guarana (*Paullinia cupana Kunth*), and/or mate (*Ilex Paraguariensis* A.St.Hil), and/or Kola (*Cola nitida* (Vent.) Schott & Endl.) to the product resulting from the drying step.

10. A method for obtaining a composition with lipolytic activity according to any of claims 8 or 9, wherein the extraction of anthocyanins and phenolic acids of blood orange (*Citrus sinensis L. Osbeck*) and sweet orange (*Citrus sinensis L. Osbeck*) juice, and the extraction of flavanones of blood orange (*Citrus sinensis L. Osbeck),* grapefruit (*Citrus paradisi Macfad.)* sweet orange (*Citrus sinensis L. Osbeck*) and bitter orange (*Citrus aurantium L.*) by-products are proceeded to.

11. A method for obtaining a composition with lipolytic activity according to one of claims 8 to 10, wherein prior to the drying step is carried out a step of removing water-insoluble particles from the mixture.

12. Use of the composition with lipolytic activity according to claims 1 to 7 as a food supplement.

13. Use of the composition with lipolytic activity according to claims 1 to 7 for enriching foods, namely beverages such as fruit and/or vegetable juices as well as dairy products.

14. Non-therapeutic use of the composition with lipolytic activity according to claims 1 to 7 for administration in order to promote fat burning.

15. Non-therapeutic use of the composition with lipolytic activity according to claims 1 to 7 for administration in order to promote loss of body weight.

16. Non-therapeutic use of the composition with lipolytic activity according to claims 1 to 7 for its lipolytic activity administration at a dosage varying between 1.0 and 2.0 grams, preferably of 1.4 grams of composition a day.
